# EUROPEAN PATENT APPLICATION

(11) **EP 2 478 868 A1**
(43) Date of publication of application: **25.07.2012**
(21) Application number: 11152133.2
(22) Date of filing: 25.01.2011
(51) Int. Cl.: A61F 2/24

(54) **Implant device**

(71) Applicant: The Provost, Fellows, Foundation Scholars, and the other Members of Board, of the College of the Holy and Undivided Trinity of Queen Elizabeth, Dublin 2 (IE); National University of Ireland Galway, Galway (IE)
(72) Inventor: Murphy, Bruce, Co. Dublin (IE); Crowley, James, Co. Galway (IE); Breen, Liam, Co. Limerick (IE); Early, Michael, Co. Waterford (IE)
(74) Representative: Lane, Cathal Michael

(57) **Abstract**

An implant device for a heart having a first chamber and a second chamber and a native valve between the chambers, the device having an elongate body having opposing ends, the body being configured to be implanted within the heart and extend through the native valve with a first end in the first chamber and a second end in the second chamber; a first resiliently compressible part of the body being adapted to be compressively engaged by the heart within the first chamber; and a second resiliently compressible part of the body being adapted to be compressively engaged by the heart within the second chamber; so that the first and second parts hold the elongate body in a desired position within the heart by said compressive engagement. The device can be employed as a prosthetic device replace or supplement a native valve such as the mitral valve.

## Description

### Field of the Invention

The present invention relates to implant devices for the heart and valves and in particular native valves within the human or animal body. Of particular interest are heart valves. The device of the invention is for use with valves that are dysfunctional and in particular for use with valves which do not close properly or otherwise allow blood flow through the valve when closed, for example due to perforation. In other cases the valve may close in a correct manner but the annulus across which it closes is dysfunctional, for example it may be dilated. Such dysfunctional valves are often referred to as leaky/leaking valves. Valves that do not close properly impair the pumping function of the heart as blood pumped by the heart can leak back through the valve instead of being pumped out through the vasculature. The present invention is particularly useful for dysfunctional mitral valves. While the present invention is described in relation to the mitral valve it will be appreciated that it is intended for application to other valves such as the tricuspid valves.

### Background to the Invention

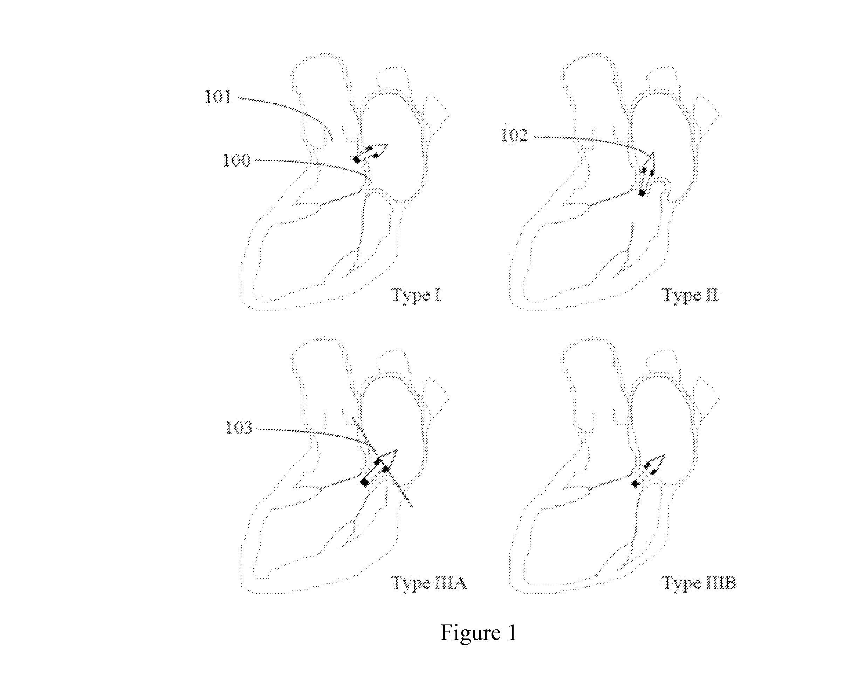

There are a number of states of mitral valve dysfunction that can cause regurgitation of blood into the left atrium. (Often referred to as mitral regurgitation or "MR"). **Figure 1** below illustrates four different defects (Type I - Type IIIb) as classified under the Carpentier classification for mitral valve disease. The four figures of this schematic all have one common result (because of failure of the mitral valve 100 to close properly): during contraction of the left ventricle, blood is not optimally pumped through the aortic valve 101, as this contraction also causes blood to regurgitate into the left atrium. The regurgitation flow is shown by arrow 102 in each of the Figures. The plane of the mitral valve annulus is indicated by the dashed line indicated by reference numeral 103. Type I is characterized by normal leaflet length and motion but with either annular dilation or leaflet perforation, such as with endocarditis. Type II MR is caused by leaflet prolapse, usually from myxomatous disease, or by papillary muscle rupture or elongation. Type III MR is caused by restricted leaflet motion. Type IIIa is classically caused by rheumatic disease with subvalvular involvement. Type IIIb is typically caused by ischemic or idiopathic cardiomyopathy with ventricular dilation causing tethering and restricted motion of the leaflets. (See for example Cohn LH, Edmunds LH Jr, eds. Cardiac Surgery in the Adult. New York, NY: McGraw-Hill; 2003:987-997.)

Patients with a dysfunctional mitral valve (the heart valve between the left ventricle and left atrium) are usually treated by surgical repair/replacement of the valve which is a major invasive surgery. However, in up to 55.7% (see: Circulation 2008; 117: 963-974) of cases these patients are left un-treated as they are deemed unsuitable candidates for such an invasive surgery. If the current surgical procedure can be reduced to a successful minimally invasive procedure it potentially would deal with this inoperable patient cohort. There is thus a problem to be solved with providing a solution for patients on whom invasive surgery cannot be carried out to repair such defects.

The company Evalve which is part of Abbott Laboratories sell a device under the brand name **Mitraclip™**. The device takes the form of a double-leg clip each leg being openable and closeable. It can be delivered using minimally invasive techniques through the venous system. It is passed through the septum wall into the left side of the heart. Repeated opening and closing of both sides of the clip is required because each side must capture either the anterior or posterior leaflet of the mitral valve while it is opening or closing with the natural heart rhythm. Clipping both leaflets at the correct location on the first attempt is difficult and multiple locating steps are generally required. This is surgically very time-consuming (up to 6 hours) and this difficulty implanting the device remains a very serious drawback. Once in place the clip stops valve leaflet prolapse and inhibits mitral regurgitation. However, because of the construction of the device it must be delivered with a 26F delivery catheter (8.66 mm diameter). Large delivery catheters can cause complications including tearing within the vasculature, for example dissection of the femoral vein. Because of the necessity to pass the device across the septum of the heart, large holes will remain in the heart wall in addition to a large hole at the venous access site. Potentially this gives rise to future potential complications. The device is based on a surgical technique that was only moderately successful and achieved its highest success rates only when combined with annuloplasty and thus may not achieve good long-term clinical results.

The Edwards Lifesciences company has a system called Monarc™ which has a flexible rod forming a bridge between two annular self-expanding stent-like matrices. It can be delivered in a collapsed state by a catheter and expands when deployed. One (larger) annular matrix forms a proximal anchor and the second (smaller one) a distal anchor. The device is placed in the coronary sinus vessel. The coronary sinus is sufficiently proximate the (periphery of) mitral valve annulus so that force applied to the coronary sinus transmits to the annulus. The bridge part of the device progressively shortens as a biodegradable element dissolves imparting a greater tension which pulls the coronary sinus and the mitral valve annulus inwards. This has the effect of drawing the two leaflets closer together which limits the amount of mitral regurgitation. This is designed to deliver the desired correction to the posterior mitral annulus by curving the coronary sinus to a radius that achieves an effect similar to that achieved with an annuloplasty ring.

A similar system is sold by the Cardiac Dimensions company under the trade name Carrilion™. It too has a dual anchor system with a bridging rod between two expandable anchors and uses force applied to the coronary sinus to impart a correcting force to the mitral annulus. A further coronary sinus device is sold by the company Viacor under the brand name PTMA™. It consists of inserts with an implanted flexible tube, the inserts imparting a desired shape to the coronary sinus and thus the mitral valve annulus.

US Patent No. US 7,291,168 describes an open frame or cage structure implant which resides entirely within a left atrium of the heart and above the mitral valve. The implant engages an upper wall of the atrium and interacts with leaflets of the mitral valve. A similar arrangement is shown in related International (PCT) patent publication WO 03/028558 and US Patent Publication Nos. US 2004/00138745 **and** US 2008/0140190**.** An open frame or cage structure which works in a similar fashion is described in International (PCT) patent publication WO 2005/007036**.**

A device with a frame structure is described in US Patent Publication No. US2006/0106456 which is fixed by suture or may be held by pressure exerted between the valve and the device. A similar device is described in US Patent Nos. US 7,381,220**,** and US Patent Publication No. US 2008/0065204**.**

International (PCT) patent publication No. WO 2007/025028 and related US Patent No. 7,611,534 describe a cage structure which fills an arterial chamber and which has an expandable ring to engage the annulus of a heart valve. A similar structure is shown in US Patent Publication No. US 2008/0033541**.** US Patent Publication No. 2006/0058871 (and related WO 2006/032051**)** shows a cage structure with a pocket. The pocket expands and contracts with blood flow to block leakage of the mitral valve when expanded and to cause minimum blood flow disruption when contracted.

US Patent Publication No. US 2007/0265700 describes a heart implant device with an elongate shaft. The implant is tethered to the wall of the left ventricle, by an anchor, which is surgically inserted into the wall. It extends though the mitral valve and has a spacer which is located proximate the mitral valve to interact or replace the valve. Safety stops may also be provided to restrict unwanted movement of the device. A similar arrangement is shown in US Patent Publication No. US 2007/0270943**.**

US Patent Publication No. US 2003/0083742 describes a valve repair device which comprises a ring, a stem and a connector for connecting to valves for the heart. The stem is placed through a surgical cut in the annular attachment.

US Patent Publication No. US 2009/0149949 (and related patent documents WO2006/064490**,** US2006/0178700 and US2009/0076600**)** describes a device which comprises a support and treatment element which supports valve leaflets. One end of the device is attached to a wall of the heart by an anchor which is secured in the wall of the heart. The device extends through the valve and it has a plug thereon for occluding leakage that may occur between the valves.

A similar device that is intended for use with the tricuspid valve is described in US Patent Publication No. 2006/0241745 (and related publication WO 2006/111391**).** The device is anchored by an anchor inserted into a wall of the heart within the right ventricle and is anchored at a second end by a stent like anchor that is expanded within the superior vena cava.

One of the issues with the prior art described above is the need for exact surgical positioning of the device. In particular most devices require not only great skill to get them to the site within the heart, but they also require great precision to fix them in place to ensure they do not move after implantation. Such precision is difficult with a moving heart muscle.

### Summary of the Invention

In one aspect, the present invention provides a minimally invasive technique and device that can be implanted in the heart It can be used to repair valve dysfunction for example mitral valve dysfunction.

The invention provides an implant device for a heart having a first chamber and a second chamber and a native valve between the chambers, the device comprising:
(a) an elongate body having opposing ends, the body being configured to be implanted within the heart and extend through the native valve with a first end in the first chamber and a second end in the second chamber;
(b) a first resiliently compressible part of the body being adapted to be compressively engaged by the heart within the first chamber; and
(c) a second resiliently compressible part of the body being adapted to be compressively engaged by the heart within the second chamber; so that the first and second parts hold the elongate body in a desired position within the heart by said compressive engagement.

The device of the invention is simple and can remain in place under the compressive force once delivered and positioned. This is a simple yet highly effective arrangement which allows for positioning of the device within the heart for replacement or repair of a valve. The device is much more easily placed within the heart than conventional devices which typically require anchoring/suturing to the heart wall before they are initially positioned immediately after delivery.

Desirably the main compressive force holding the device in place is an axial compressive load. The load is imparted when fitted within the heart, for example between the apices of opposing cavities within the heart. It is however desirable that the device of the invention is dimensioned to seat itself within the heart at a position where it does not abut the apex of the cavity wall, in particular the ventricle. It is desirably held a distance away from the apex (i.e. short of the apex position) by wedging (radial gripping) by opposing heart walls. The heart wall at the apex is not so strong as other parts of the heart so this arrangement is thought to provide less risk of puncture or other damage to the heart wall. The compression is transmitted axially along the elongate body so the elongate body will be adapted to withstand sufficient force so as to allow it to hold the first and second resiliently compressible parts in position in their respective chambers.

Desirably the device of the invention further comprises at least one treatment element for treating the heart. The treatment device will typically be for restoring some impaired function of the heart, for example to repair or replace a valve, or to occlude an aperture, or to reduce the volume of a chamber of the heart, for example where the heart has enlarged and the effectiveness of pumping of the heart has been reduced by enlargement of a chamber of the heart. For example the treatment element could be a valve member or a volume-reducing component for reducing the active volume of a chamber of the heart. Desirably at least one of the first part or the second part comprises a series of struts. Desirably at least one of the first part or the second part comprises a compressible lattice structure. Desirably the shape of the first or second part has a curved outer profile for example a periphery that forms a loop about a longitudinal axis of the elongate body.

For example the device may comprise a ring that extends about an axial axis of the device. An at least part spherical shape, may be employed.

In one arrangement at least one of the first part or the second part comprises at least two leaves which are resiliently biased part and are adapted to be compressively engaged by the heart. In this case compression brings the leaves closer together against the resilient bias. For example the leaves may be opposing arms that respectively engage against opposing internal walls of the heart. Desirably the leaves are curved, for example in cupped shape such as a partial C-shape. As the heart contracts the leaves move toward (systole phase of rhythm) and away (diastole phase of rhythm) from each other.

Desirably at least one of the first part or the second part comprises a compressible structure such as a cage structure adapted to be compressively engaged by the heart. The cage can be formed in any desired shape, for example in a bulbous shape, as a loop or band or as a curved web of material, for example in a general basket shape. The cage can be formed with a substantially closed form for example a bulbous shape. Or it may be formed by a ring that is open top and/or bottom, for example a band of lattice material. It is desirably profiled for minimum potential damage to the heart. For example its outer profile may be tapered and/or curved.

More than one compressible structure may be provided for any given chamber of the heart. For example two or three cage structures could be provided for a chamber of the heart, for example the atrium chamber.

It will be appreciated that where at least one of the first part or the second part is mentioned, it may be desirable to have both with the same structure.

For different chambers of the heart the first and second portions may be differently shaped and sized for example to account for the anatomy of the heart, whether or not they have substantially the same construction.

Struts forming the first and/or second apart may be configured in a mesh arrangement and be provided in any desirable shape/form as discussed herein.

Desirably the elongate body is collapsible to a delivery configuration. This means it can be delivered by conventional means such as via a catheter.

The device of the invention may be formed by cutting the body from a tubular element, and/or from at least one braided filament. It will be appreciated that any suitable method of manufacture can be used. Nitinol and other suitable materials may be used.

Suitably the first part of the body is dimensioned to substantially occupy a chamber of the heart. Alternatively or additionally the second part of the body is dimensioned to substantially occupy a chamber of the heart. In this way they are easily locatable within the heart.

Desirably at least one of the first and second parts, and desirably both, are in the form of a cage, and in particular an end cage at each end of the device. (It will be appreciated that the end cages may be adapted to hold the device away from the apex of the heart, and it is desirable to have the device adapted in this way particularly for any cage which is for use in the ventricle.

It will thus be appreciated that the first and second parts may also experience a radially compressive load. This may in particular be the case if the first or second part is larger than the cavity within the heart into which it is fitted, for example larger than the apex of the cavity in which it is placed, for example the apex of the ventricle. The compressive load may also be applied when the heart contracts during the heartbeat cycle, for example upon contraction of the ventricle. In such a case the load applied to the device will be a combination of both an axial and a radial compressive force.

Desirably the device comprises a valve member which is arranged to improve the overall valve function of the heart. For example the valve member may be selected from one or more of: a replacement valve for replacing the native valve; a replacement leaflet for replacing a leaflet of the native valve such as the anterior or posterior leaflet; a supplementary valve for supplementing the native valve; a stop member for restricting movement of at least one leaflet of the native valve; or an occlusion member for occluding an aperture in a valve or a gap not closed by the valves. For example the device may be arranged to prevent prolapse of one or more valve leaflets of the heart. The stop member may take the form of a plate. It will be appreciated that the device of the invention may include more than one of such valve members in combination including a plurality of any given type.

Desirably the valve member is collapsible for delivery of the device. For example it may be resiliently compressible. The valve member is resiliently compressible so that it is compressed by the native valve. In such an arrangement it may for example form an occluding member such as one that closes all or some part of the valve, for example when the valve closes about it. In such an arrangement the valve member is dimensioned so as to be under radial compression by the native valve when in place in the heart. It may for example be squeezed inwardly in a substantially radial direction during the closing action of the valve (and released again with the opening action).

The valve member may further comprise an occluding peripheral skirt for occlusion of blood flow about the valve member in at least one direction. The peripheral skirt may sit about the annulus of the valve. For example retrograde flow within the heart may be alleviated in such a manner. Where the device of the invention functions as a replacement valve for the natural valve it is desirable that the valve member further comprises replaceable leaflets.

Desirably the first part is formed by at least one closed loop, for example a filament turned back upon itself. This is a simple yet compressible structure. Desirably the first part is formed by at least four closed loops. The second part may additionally or alternatively be formed by at least one closed loop and desirably the second part is formed by at least four closed loops. Use of curved surfaces such as loops in this way prevents angular edges or corners that could puncture or otherwise damage the heart. Use of elongate curved surfaces, for example multiple loops, can spread the load over a much wider area. The loops can take the formation of a cage structure as mentioned above.

It is desirable that a device of the invention further comprises a cushioning barrier for cushioning between the device and heart tissue. This is particularly the case where the device would otherwise abut the heart directly. The cushioning barrier is desirably a cushioning pad or the like shaped to a desired fit.

It is desirable that a device of the invention further comprises a tissue integration material attached to at least one of the first or second parts and adapted to promote tissue growth over the device. Integration of the device of the invention into tissue over time is desirable to allow it to stay in place.

The cushioning barrier may be formed by tissue integration material adapted to promote tissue growth over the device. One or both of the cushioning barrier and the tissue integration material may be a fibrous material. The cushioning barrier and/or tissue integration material may be a polymeric material, for example a fibrous polymeric material. Either or both can additionally form a volume-reducing component for reducing the active volume of a chamber of the heart.

A device of the invention may include a volume-reducing component for reducing the active volume of a chamber of the heart. The device can thus reduce the volume of blood in a chamber without actually reducing the amount of tissue of the heart or otherwise changing the heart. Typically the volume-reducing component is a barrier that occludes part of a chamber thus reducing the effective working volume of the chamber without actually changing the chamber itself. It will be appreciated that the volume-reducing component need not necessarily completely seal off a part of a chamber but instead it may form a substantial barrier to blood flow to the part of the chamber occluded. It may simply form a desirable partition. The barrier may take any suitable form for example that of a sheet. Such a device is particularly suited to hearts that are enlarged, for example a heart with an enlarged ventricle. Reducing the volume allows for more efficient pumping of the heart because the active volume of the chamber is once again of a size commensurate with the pumping capability of the heart. With enlarged chambers the volume of blood is greater than can be pumped by the heart. The volume-reducing component can be used on a device of the invention with or without the added functionality of a valve member. When a volume-reducing component and a valve member are utilised together they can provider more efficient pumping and better valve functionality thus providing cumulative improvement in heart function. Like all other parts of the device it is desirable that the volume-reducing component is compressible to allow it (together with the rest of the device) to be delivered by catheter. It desirably takes the form of a partition membrane. It may be flat or concave with a sheath type arrangement.

As mentioned above fixing prior art devices in place typically consists of inserting one or more sharp/barbed hooks into the tissue of the heart at a desired location. Alternatively the devices may be sutured in place. The entire load on such conventional devices is borne in a tensive arrangement where the device is under tensive load from the hooks or sutures.

The device of the invention can be deployed in its correct location and does not require any anchoring to initially position it in a desired position. It does not require any attachment to the heart, for example by insertion of a securing means into heart tissue to initially position it. It can, at least initially, stay in place by non - penetrative abutment of opposing parts of the heart.

However it is still desirable that the device remains in its location after initial positioning. This is in particular true of the necessity to prevent movement such as rotation of the device over time. Movement of the device over time is indeed likely due to the repetitive movement of the heart. In such a case it is desirable that a device of the invention further comprises one or more barbs for anchoring the device to the heart. The barbs may be on the device on at least one of: the first part, the second part or at a position on or proximate the valve member. For example the barbs may be used to anchor the device to the native valve for example to at least one of a leaflet or an annulus of the native valve.

The device of the present invention is dimensioned to undergo axial compression to effectively wedge itself in place between opposing locations on the heart - for example at the top of the left atrium and the bottom of the left ventricle. The axial compression may be along its entire length between opposing ends thereof.

The device of the invention may be utilised with patients that are not otherwise suitable for surgery required to carry out repairs, and thus represents a good alternative to surgery.

The device of the invention will desirably be resiliently compressible both axially and radially. This will ensure that when implanted it stays in place. Also it will be sufficiently radially compliant so that it compresses under forces imparted by the heart and does not interfere with contraction of the heart.

The device of the invention can be formed with a damper arrangement for at least partially absorbing compressive forces in an axial direction.

The invention also provides an implant device for a heart having a first chamber and a second chamber and a native valve comprising native leaflets between the chambers, the device comprising:
an elongate body having opposing ends, the body being configured to be implanted within the heart and extend through the native valve with a first end in the first chamber and a second end in the second chamber,
wherein the device is configured to replace one leaflet of the native valve which leaflet is adapted to work in cooperation with a remaining leaflet of the native valve to achieve native valve-like functionality.

The replacement leaflet may comprise a prosthetic tendon that acts in a manner analogous to a native tendon for a native leaflet.

The present invention thus provides: a device for positioning a valve member in the heart proximate a native heart valve, the native valve for closing an aperture to separate two chambers of the heart the device comprising:
an elongate body having opposing ends (along a longitudinal axis of the body),
the body for extending through the native valve,
a first part of the body being dimensioned to fit within a first chamber;
a second part of the body for locating within the second chamber
wherein the first and second parts are arranged to position the body in place with the valve member proximate the native valve by compressively engaging its opposing ends within the heart.

The device of the invention can be considered to be a device for positioning a valve member in the heart proximate a native heart valve, the native valve for closing an aperture within the heart the device comprising:
an elongate body for extending through the valve,
a first part of the body being dimensioned to be gripped between upper side walls of the heart;
a second part being dimensioned to be gripped between lower side walls of the heart;
wherein the first and second parts are arranged to position the body within the heart with the valve member proximate the native valve by engaging in an axially compressive arrangement between the upper and lower side walls.

The invention can be considered to be a device for positioning a valve member in the heart proximate a native heart valve, the native valve for closing an aperture to separate two chambers of the heart the device comprising:
an elongate body for extending through the valve,
a first part of the body being dimensioned to fit within a first chamber;
a second part for locating within the second chamber
wherein the first and second parts each compress and expand to conform to a shape imparted by a heart wall under force of the pumping cycle of the heart.

The features of the present invention have been described above. It will be appreciated that all possible combinations of the various arrangements set out above are contemplated within the present invention.

The invention extends to a device substantially as described herein with reference to and/or as illustrated in the accompanying drawings.

### Brief Description of the Drawings

Embodiments of the invention will be described, by way of example only, with reference to the accompanying drawings in which:

**Figure 1** is a schematic representation of a cross-section of the heart;

**Figure 2A** is a schematic representation of a device of the invention in the form of a mitral valve treatment prosthesis;

**Figure 2B** is a schematic representation of the device of Figure 2A with a valve member in the form of a replacement valve attached thereto;

**Figure 2C** is a schematic representation of the device of Figure 2A implanted within a human heart;

**Figure 3A and Figure 3B** are schematic representations of cage structures suitable for forming a first or second part of a device of the invention;

**Figure 4A** is a schematic representation of a device of the invention with two separate resiliently compressible parts of the body each in the form of a cage structure and designed to be placed in one chamber;

**Figure 4B** is a schematic representation of a device of the invention with three separate resiliently compressible parts of the body each in the form of a cage structure and designed to be placed in one chamber;

**Figure 5** is a series of images (labelled **A-D)** showing a prototype device similar to that of Figure 2A being inserted into a delivery catheter;

**Figure 6** shows a schematic representation of a substrate from which a device of the invention has been partially formed;

**Figure 7** shows an image of a partially formed device of the invention being constructed utilising a filament being worked onto a shaped former;

**Figure 8** is a series of schematic representations **(A-G)** showing an illustrative cross-section of devices of the invention implanted in a heart by way of showing the position relative to the plane of the mitral valve;

**Figure 9** is a series of schematic representations **(A-H)** showing various views of a valve member in the form of replacement valve in open and shut configurations;

**Figure 10** is a schematic representation of a device similar to that shown in Figure 2A and 2B being inserted through the native valve of the heart;

**Figure 11** shows various views of a replaceable valve embodiment of the present invention;

**Figure 12** shows a schematic representation of a device similar to that shown in Figure 2A and 2B with an occlusion member as a peripheral skirt for occluding a leak in the valve;

**Figure 13** shows a schematic representation of a device similar to that shown in Figure 2A and 2B having a prolapse preventing member thereon and inserted in the heart;

**Figure 14** shows a schematic representation of a device similar to that shown in Figure 2A and 2B wherein the device is configured to be in an offset position where it extends through the native valve at a position offset to one side of the valve;

**Figure 15** is a perspective view of a device similar to that shown in Figure 13 further including a damper/spring arrangement for axial compression of the device;

**Figure 16** is a schematic representation of a device of the present invention similar to that of earlier Figures showing in **Figure 16A** a compressed configuration of the lower part 21 during left ventricle systole and in **Figure 16B** an expanding configuration during diastole;

**Figure 17** is a schematic representation of a device 1 of the present invention similar to that of earlier Figures with different resiliently compressible parts;

**Figure 18** is a perspective view of a device of the invention that is similar to that of Figure 17 but with a different resiliently compressible upper part;

**Figure 19** is a sectional view of a device of Figure 18 additionally configured as a plugging device, shown in **Figure 19A** **in** systole and **Figure 19B** in diastole;

**Figure 20** is a sectional view of a device of Figure 18 additionally configured with a supplementing prosthetic valve, shown in **Figure 20A** in systole and **Figure 20B** in diastole;

**Figure 21** is a sectional view of a device of Figure 18 additionally configured with a supplementing valve leaflet, shown in **Figure 21A** in systole and **Figure 21** **B in** diastole;

**Figures 22 A-D** are schematic representations of structures suitable for forming a first or second compressible part of a device of the invention;

**Figure 23** shows a device of the invention in an arrangement very similar to that of Figure 10 and with cushioning buffers or barriers on the device;

**Figure 24** shows a device of the invention in an arrangement very similar to that of Figure 10 and with barbs attached to the device at a position which engages the mitral valve; and

**Figure 25** is a schematic representation of a device of the invention in the form of a prosthesis for reducing the volume of a chamber of the heart.

It is to be noted that the Figures are schematic and are not drawn to scale and different scales are used for ease of illustration.

### Detailed Description of the Drawings

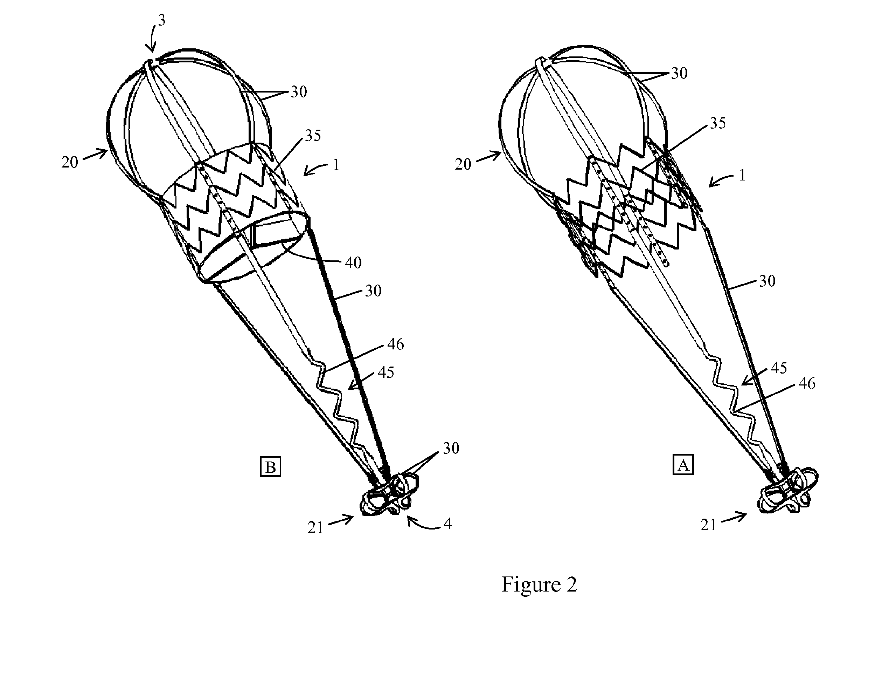

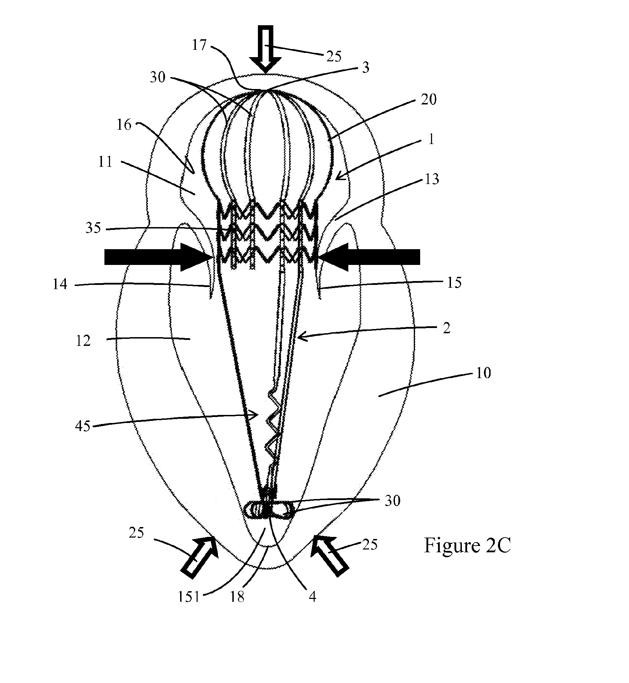

**Figure 1** has been described above. **Figure 2A** is a schematic representation of a device 1 of the invention in the form of a mitral valve treatment prosthesis. **Figure 2B** shows the device of Figure 2A with a treatment element in the form of a valve member in the form of a replacement valve attached thereto and **Figure** 2C shows it implanted within a human heart 10. (A simplified heart is shown for the purposes of illustration.) The heart 10 has a first chamber (atrium) 11 and a second chamber (ventricle) 12 and the native mitral valve 13 between the chambers. The mitral valve 13 has two leaflets 14;15.

As with all embodiments the device 1 is collapsible (by being resiliently compressible) to a delivery configuration. This means it can be delivered by conventional means such as via a catheter as will be described in detail below.

The device has an elongate body 2 with opposing ends, top end 3 and lower end 4. The body 2 is being configured to extend through the mitral valve 13. The top end 3 in atrium chamber 11 and the lower end 4 is in the second chamber. The device 1 has a first resiliently compressible part of the body in the form of a cage structure with a bulbous shape 20 is compressively engaged by the inner heart wall 16 within the chamber 11. A second resiliently compressible part of the body in the form of a smaller cage structure in a bulbous shape 21 is compressively engaged by the heart within the second chamber 12. The device 1 is thus held in place by said compressive engagement. The entire device 1 can compress and expand with the movement of the heart. So it is squeezed with subsequent squeezing pressure release repetitively during the heart cycle.

As indicated by the arrows labelled with reference numeral 25 the main compressive force holding the device 1 in place is an axial compressive load along the body 2. The load is imparted to the device 1 by the heart, for example between the apices of opposing cavities within the heart. It is however desirable that the device of the invention is dimensioned to seat itself within the heart at a position where it does not abut the apex of a cavity wall. It is desirably held a distance away from the apex (i.e. short of the apex position) by wedging (radial gripping) by opposing heart wall portions as indicated in Figure 2C. The heart wall 16 at the apex 18 of the ventricle is not so strong as other parts of the heart (e.g. the radial ventricle wall 10 of the heart) so an arrangement where the device is held away from the apex 18 is thought to provide less risk of puncture or other damage to the heart wall 16.

In the embodiment both the first (upper) part 3 and the second (lower) part 4 comprises a series of resiliently deformable struts 30 formed by filaments of nitinol material which is interwoven or braided. Struts 30 which may be integrally formed with the first or second parts extend between the two ends holding the ends together. In this arrangement the struts 30 that extend in this manner from a part of the elongate body that is resiliently compressible also.

The device 1 of the invention may be formed with a damper arrangement for at least partially absorbing compressive forces in an axial direction. In the embodiment of Figures 2A and 2B a damper 45 is provided in the form of a series of over back portions 46 which form a structure similar to a spring with resilient bias against compression. This structure is incorporated into a strut 30. Such a damper structure can be utilised to allow (resilient) compressive shortening of the device.

A series or resiliently compressible support rings 35 are provided on the device about the longitudinal axis thereof. The support rings 35 provide additional resilience to compression on the elongate body 2 proximate the position where the valve 13 and in particular valve leaflets 14,15 exert a localised compressive force on the device during the closing action of the valve during the normal heart pump cycle.

Figure 2B shows the device with a valve member in the form of a replacement valve 40 mounted within the elongate body at a position where it can act in place of the native mitral valve 13 and its leaflets 14;15 when implanted in the position shown in Figure 2C. Suitable artificial valves are already known and their construction will not be further described here.

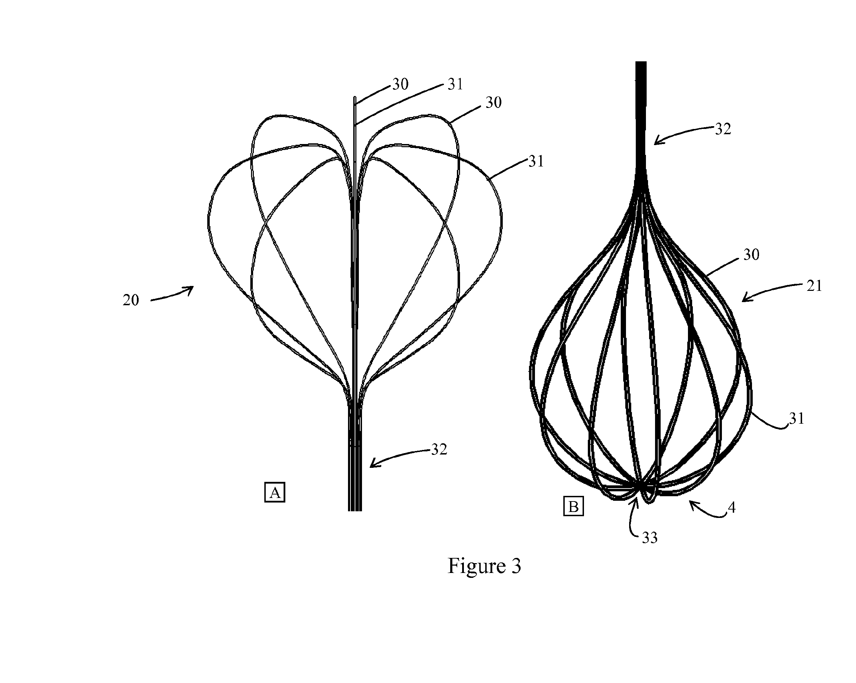

Struts forming the first and/or second part may be provided in any desirable shape/form as discussed herein and examples of which are illustrated in **Figures 3A and 3B****.** Here the struts 30 form a cage structure by virtue of forming a series of loops 31. The shape of the structures ensures there are no angular edges or sharp points for abutment with the wall 16 of the heart 10. The structures shown can form part of the device 1 and used in either chamber though it is desirable that the structure of Figure 3A is used for insertion in the atrium chamber 11 (as described above for bulbous structure 20) and the structure of Figure 3B is used in the ventricular chamber 12 (as described above for bulbous structure 21). In Figure 3A the loops 31 do not overlap being held in a back-to-back type arrangement by a central stem arrangement 32. In Figure 3B the loops 31 are also held by a central stem arrangement 32 but also converge and overlap, and are optionally held together, at an overlapping point 33 at the lower end 4.

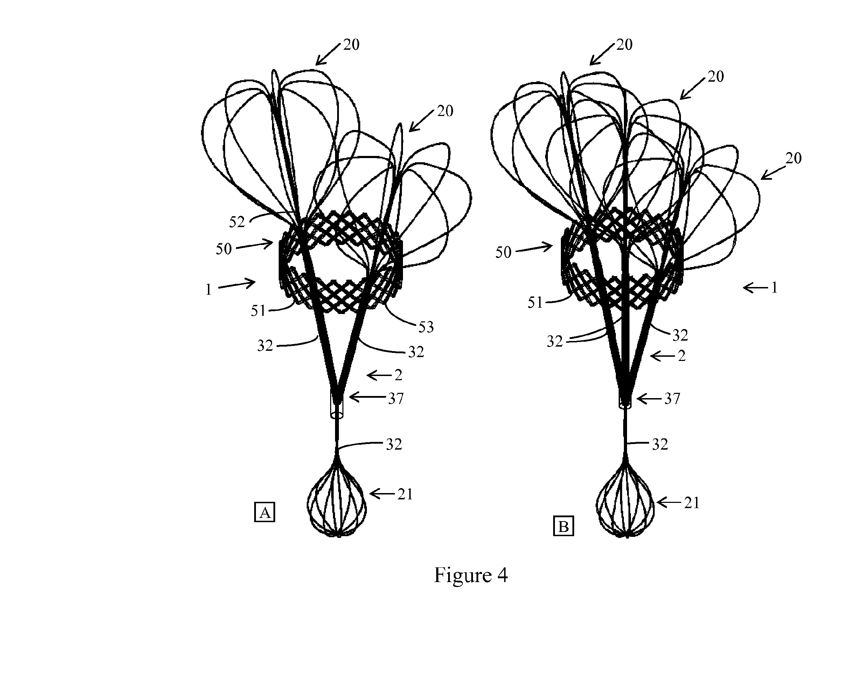

**Figure 4A** is a schematic representation of a device of the invention with two separate resiliently compressible parts of the body each in the form of a cage structure and designed to be placed in one chamber. More particularly Figure 4A shows a device 1 with an elongate body 2. The device 1 has an upper part 3 which has two cage structures 20 each similar in construction to that described above and in particular of the same construction as that of Figure 3A. The device 1 has a lower part 4 which is a cage structure 21 similar in construction to that described above and in particular of the same construction as that of Figure 3B.

In Figure 4A the elongate body 2 comprises a plurality of struts. In the embodiment there is a first strut forming the stem 32 of the cage structure 21. The two cage structures 20 each have a strut forming a stem 32 which supports each cage 20. The stems 32 are joined in a furcated arrangement at junction 37. It will be appreciated that the stems are made of resilient material and thus can be resiliently moved relative to each other (and junction 37).

A ring or band 50 of a compressible lattice structure formed by struts 51 is held on the device 1 by connection to the elongate body 2. It has a curved outer profile and a periphery that forms a loop about a longitudinal axis of the elongate body 2. The band 50 is open at its top end 52 and its bottom end 53. The band 50 is resilient compressible and can support a valve member for example in the form of a replacement valve 40 in a manner analogous to that shown in Figure 2B where reinforcing rings 35 support a valve 40.

It will be appreciated that where there is more than one structure in a given chamber of the heart each may arranged to engage with a different part of the chamber.

**Figure 4B** is a schematic representation of a device of the invention which is similar in construction to that shown in Figure 4A but with three separate resiliently compressible parts 20 of the body each in the form of a cage structure and designed to be placed in one chamber. A stem 32 supports each of compressible parts 20. This results in a trifurcated structure as shown in Figure 4B.

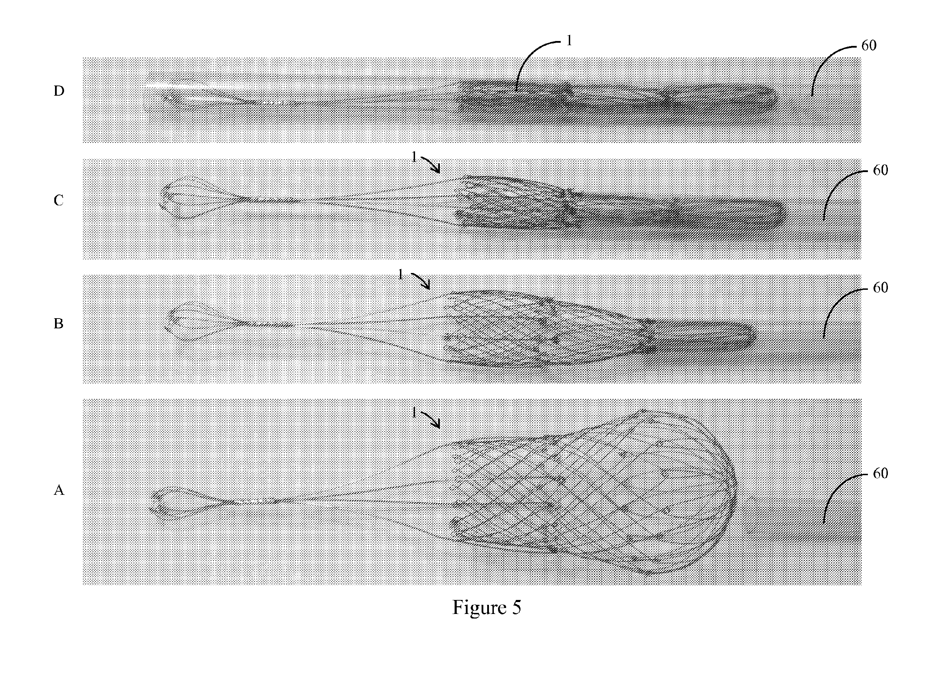

**Figure 5** is a series of images (labelled **A-D)** showing a sequence of a prototype device 1 similar to that of Figure 2A being progressively inserted into a delivery catheter 60 (the sequence is progressive from A to D). **Figure 5A** shows the device alongside the catheter 60 and before insertion; **Figure 5B and 5C** shows the device 1 in a compressed state and respectively at progressive stages of insertion into the catheter 60; **Figure 5D** shows the device 1 fully inserted in the catheter 60. The device 1 can then be delivered in its compressed state to the target site of the heart.

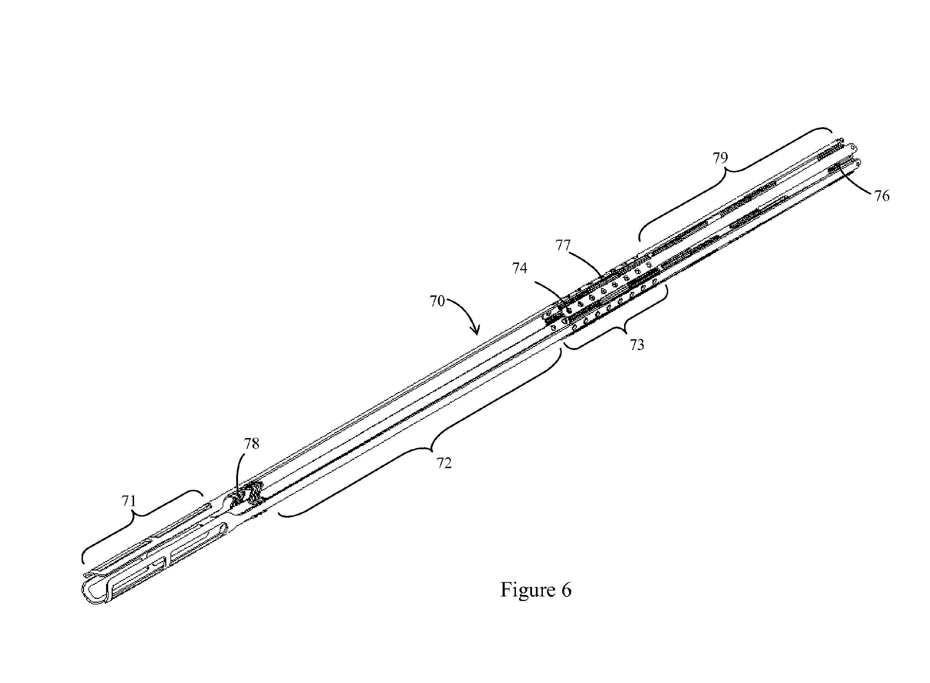

**Figure 6** shows a schematic representation of a substrate or tube 70 from which a device of the invention has been partially formed. As can be seen from the Figure the tube 60 has been worked by laser cutting to form a precursor structure of a device of the invention. Different areas of the tube form different sections of the device. For example portion 71 of the device is cut so as to form the ventricle cage. Portion 72 forms the struts 30 of Figure 2B. Portion 73 forms the support rings 35. Holes 74 located in portion 73 can be used for suturing. For example artificial leaflets may be sutured to the device using holes 74. Portion 79 forms the top end 3 of the device. Small struts 76 between the struts of portion 79 add stability to the top end 3 of the device 1. Struts 77 form the support rings 25. Struts 78 and stability to the lower end 4.

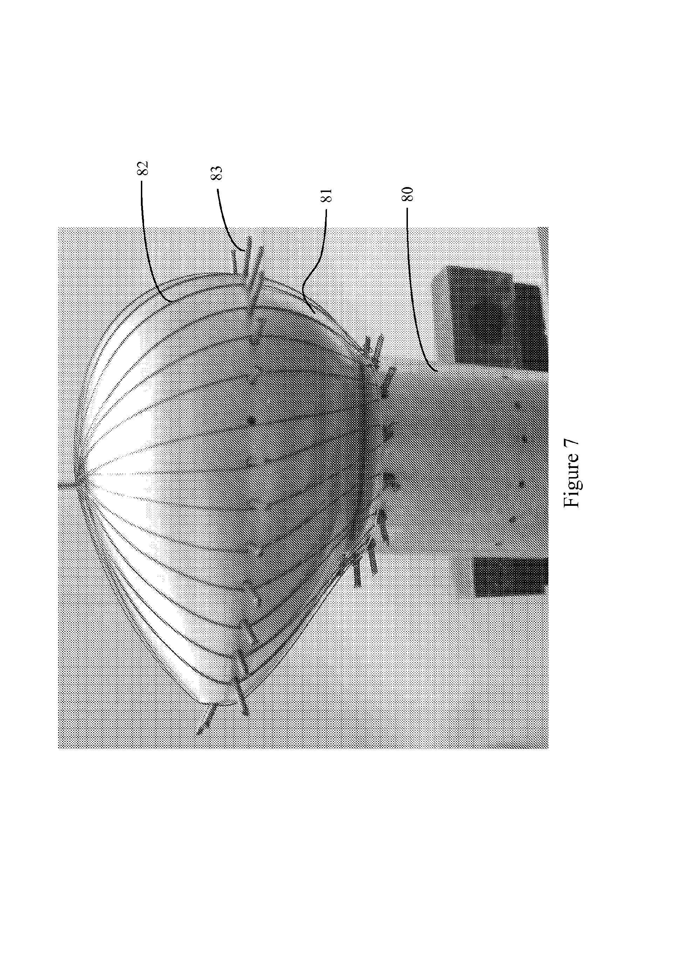

**Figure 7** shows an image of a partially formed device of the invention being constructed utilising a filament being worked onto a shaped former. A mandril 80 supports a workpiece former 81 which imparts a desired shape to a filament 82 which is worked about the former 81. Pins 83 are utilised to hold the filament in a desired position. In the image a desired cage structure is being formed. The cage being formed has an oblong or elliptical type shape. This is to match the shape of the cage to the (part of) the heart in which the cage is located. This principle can be applied even where there is more than one structure in a given chamber of the heart as each may engage with a different part of the chamber.

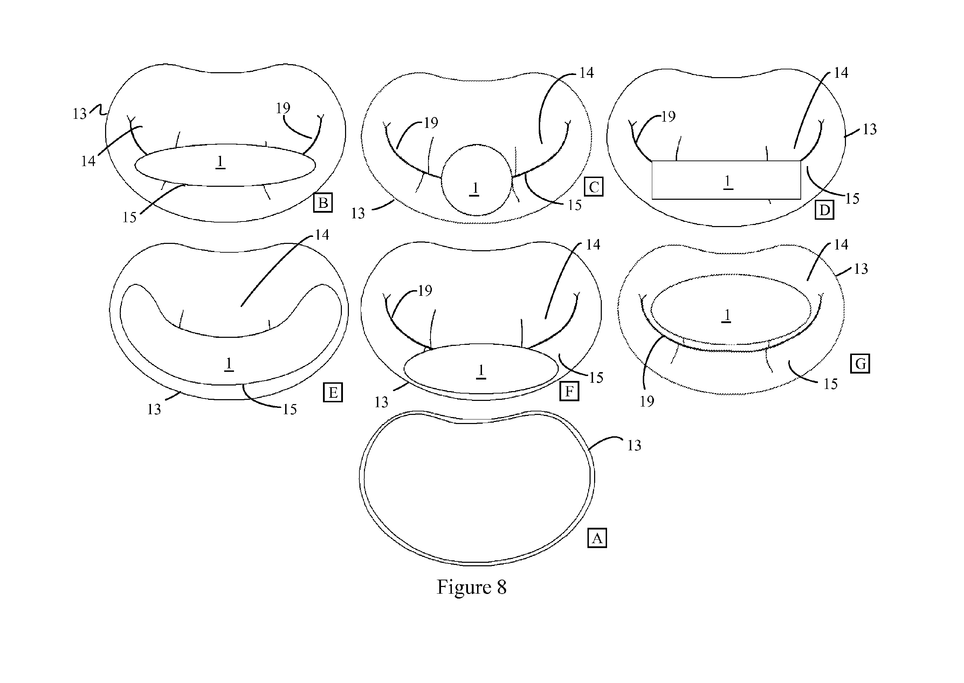

**Figure 8** is a series of schematic representations **(A-G)** showing an illustrative cross-section of devices of the invention implanted in a heart by way of showing the position relative to the plane of the mitral valve 13. **Figure 8A** shows the outline plane of a mitral valve 13. The valve 13 has leaflets 14,15. The device 1 of the invention can have varying shapes (shown in cross-section and when the heart is in ventricular systole) as illustrated and fully or partially occupy the aperture of the mitral valve by fully or partially occupying the position normally closed by one or both of the leaflets 14,15. **Figures 8B - 8D** show a device 1 centred on the junction 19 between the leaflets 14, 15 and having different sizes and shapes to compensate to differing extents for improper closing of the leaflets 14,15. **Figure 8E** shows a device 1 centred on the junction 19 between the leaflets 14, 15 and extending across the entire area occupied by the leaflets. Such a device would be used for complete replacement of the mitral valve function. **Figure 8F** shows a device 1 which is offset to one side of the valve and is substantially replacing the function of one leaflet (that is leaflet 15). In such a configuration the leaflet 14 would operate substantially as normal. **Figure 8G** shows a similar arrangement to Figure 8F but this time with a device 1 having a larger cross-section and positioned to at least partially replace the other leaflet - that is leaflet 14.

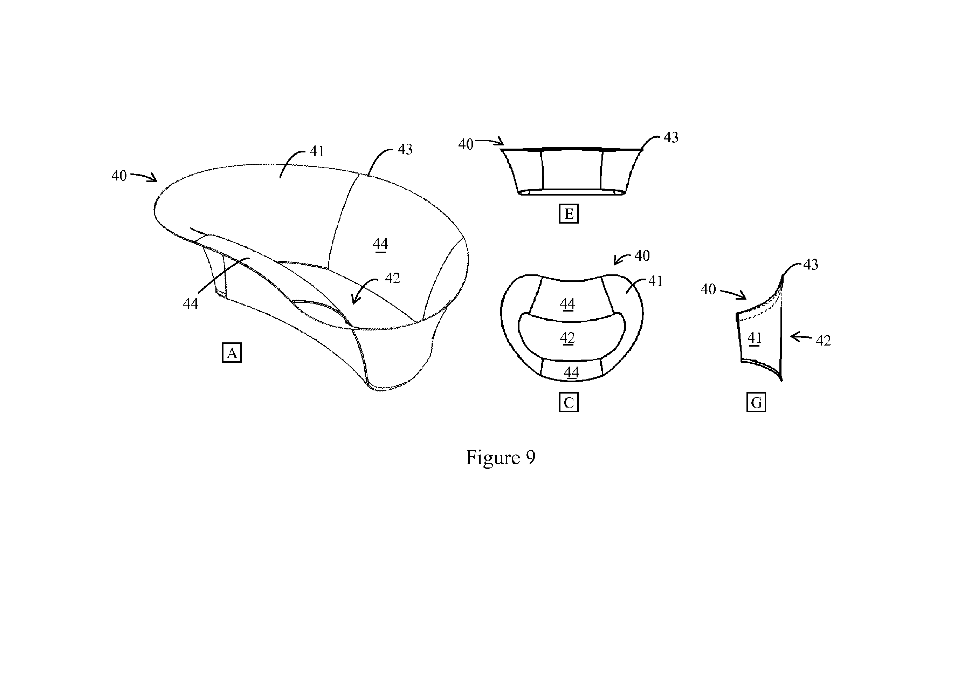

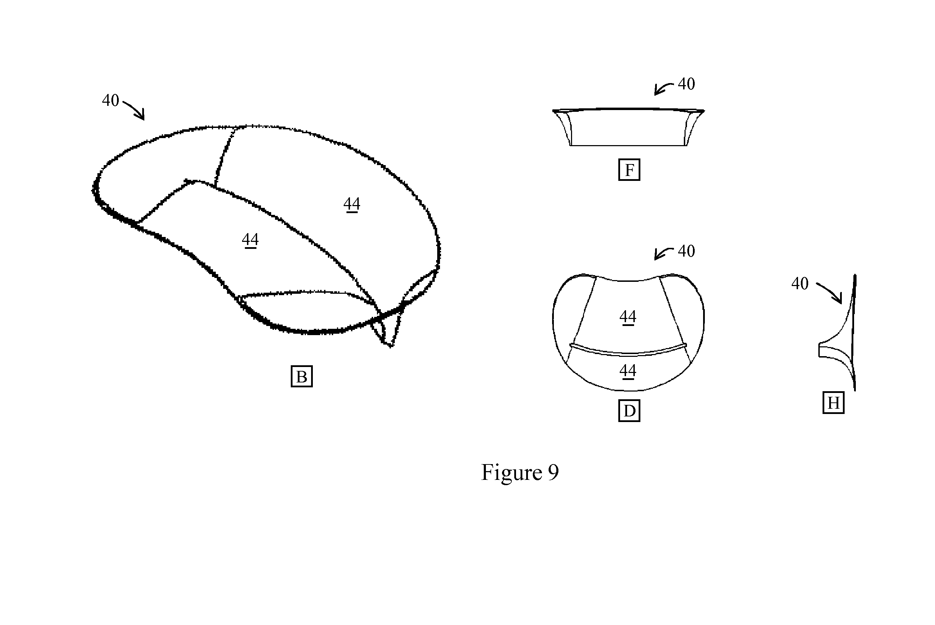

**Figure 9** is a series of schematic representations **(A-H)** showing various views of a valve member in the form of replacement valve 40 (of a type suitable for being employed with a device of the invention such as shown in Figure 2B) in open and shut configurations. **Figures 9A, 9C, 9E** and **9G** respectively show a top perspective, side elevational, top view and side view of a valve 40 in an open configuration. **Figures 9B, 9D, 9F and 9H** respectively show corresponding views with the valve 40 in a closed configuration. The valve 40 is shaped to sit at the native valve position within the heart and fulfil the valve function. It has an annular side wall 41 which is shaped to fit onto the annulus of the native valve and which defines an aperture 42. The side wall 41 has a cross-sectional shape to match that of the native valve and it slopes upwardly and outwardly terminating in a lip 43. Valve closure members or leaflets 44 can open and close with diastole and systole of the heart to mimic the valve function of a native valve.

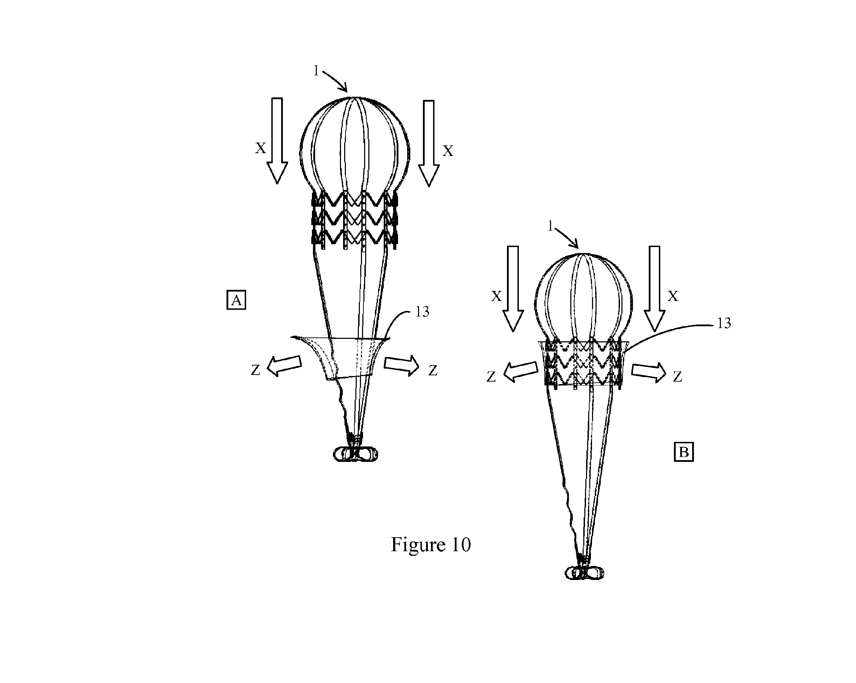

**Figure 10** is a schematic representation of a device 1 similar to that shown in Figure 2A and 2B being inserted through the native valve 13 of the heart. For the purposes of illustration the remainder of the heart is not shown.

The device 1 is inserted in through the native valve 13 progressively as shown by the sequence from **Figure 9A to 9B** using an insertion force as illustrated by the arrows labelled X. Insertion of the device 1 cause a compressive force to be exerted on the annulus of the valve 13 as indicated by arrows labelled Z. In the embodiment shown the device 1 will carry a valve member which could be a replacement valve as set out above.

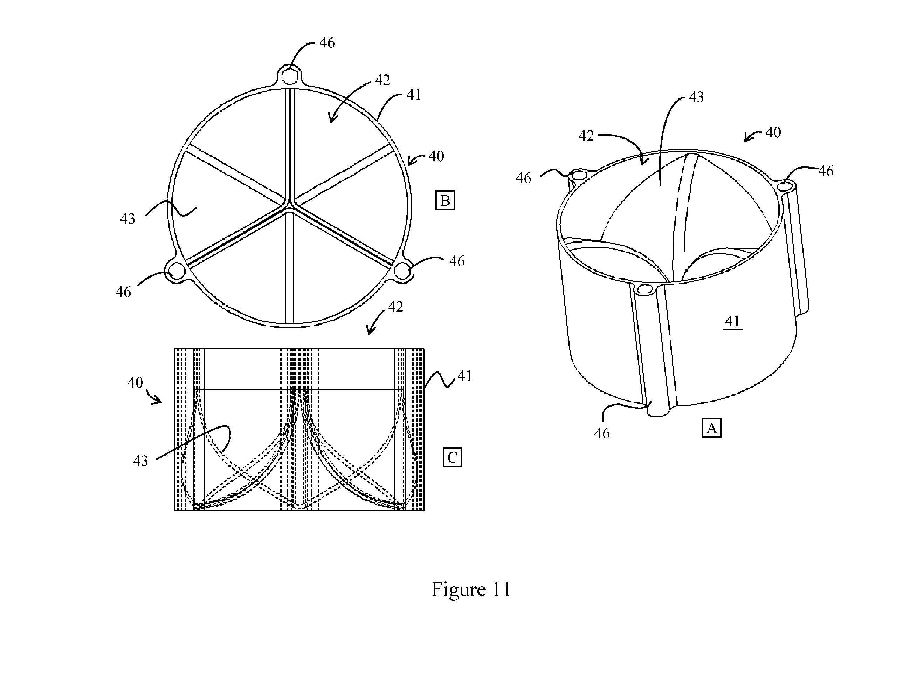

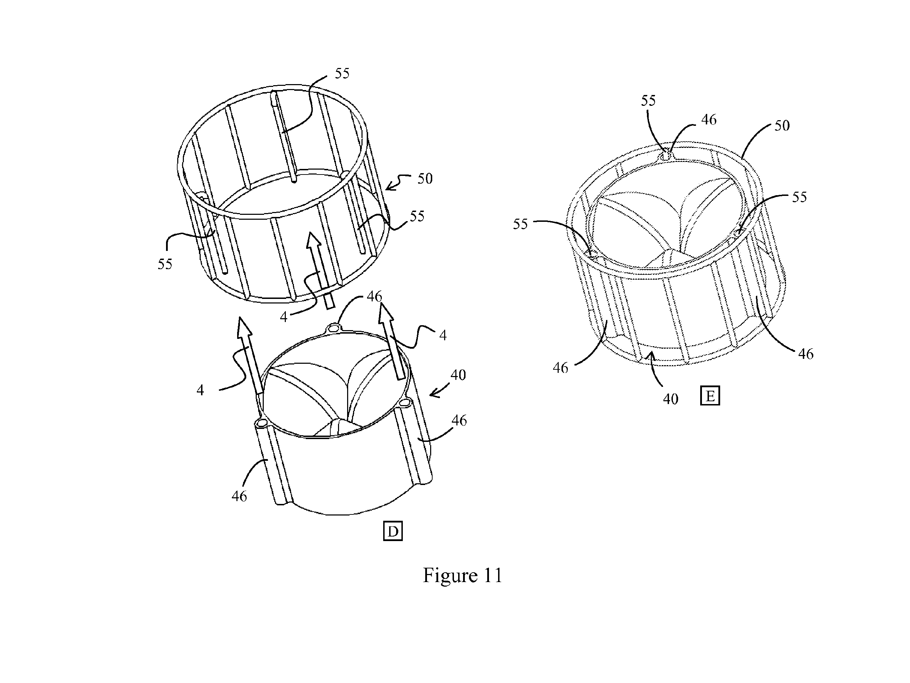

**Figure 11** shows various views of a replaceable valve 40 embodiment of the present invention. **Figure 11A-C** show respectively perspective, underneath and cross-sectional views of the valve 40, while **Figures 11D and E** show how the replacement system works. The valve 40 has an annular body 41 and a central aperture 42. Valve leaflets 43 open and close to imitate the function of the native valve. As will be appreciated the valve 40 may becomes worn out over time and accordingly it may be desirable to replace the valve 40. The present embodiment allows one to do so. In particular the valve 40 and the device 1 can be reversibly interengaged to allow the device 1 and the valve to be separated and a new valve inserted. The valve 40 is itself resiliently compressible and suitably dimensioned to be delivered (in place in a device 1 of the invention) by catheter.

The replacement sequence is best seen in Figures 11 D and 11 E where engaging means are provided on the valve 40 in the form of elongate sockets 46. A ring or band 50 is part of and held on the device 1 (the remainder of the device 1 is not shown for ease of illustration). The band 50 has the same function as that shown in Figure 4A and 4B though it is of a more simple construction. It is provided on a device 1 at a position similar to that shown in Figure 4A and 4B. Spigots 55 are formed on the band 50 and are arranged to mate with the sockets 46 on the valve 40 when aligned as indicated by arrows Y in Figure 11D. The mated assembly is illustrated in Figure 11E. If additional securing means is required to hold the valve 40 and the band 50 together in the assembled configuration any suitable means can be utilised. It will be appreciated that the assembled structure allows the valve 40 to be subsequently removed without the need to remove the device 1.

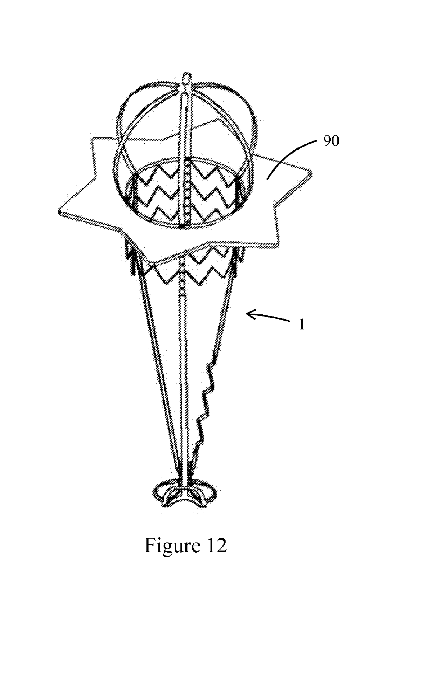

**Figure 12** shows a schematic representation of a device 1 similar to that shown in Figure 2A and 2b with an occlusion member in the form of a peripheral skirt 90 for occluding a leak in the native valve. In this embodiment the skirt forms a barrier. The skirt 90 can be of any desired shape and size and will generally be constructed of a material sufficient to block a leakage. Generally, and as shown in the embodiment, the occluding device will be arranged to sit just proximate to but above the annulus of the native valve. In this case the device will generally be utilised to completely replace the native valve leaflets. The skirt sits about the valve member and does not occlude blood flow through the valve member.

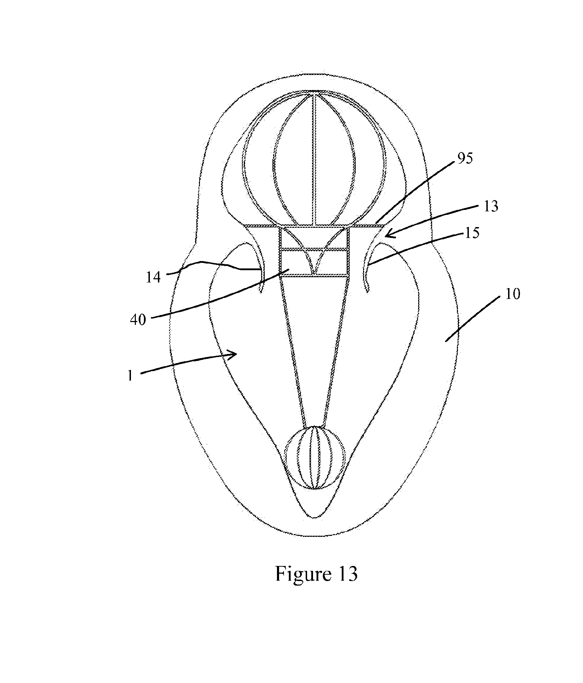

**Figure 13** shows a schematic representation of a device 1 similar to that shown in Figure 2A and 2B with a prolapse preventing member in the form of a peripheral skirt 95 inserted in the heart 10. In this embodiment the skirt 95 is formed by an open ring scaffold so that blood can flow through it. The peripheral skirt is positioned abutting the heart proximate the annulus to the native valve 13. It is adapted to prevent one or both leaflets 14,15 from prolapsing. It forms a physical barrier to the leaflet moving beyond the skirt 95 thus reducing the possibility of the leaflet prolapsing beyond its normal working position. The device 1 also includes a valve member 40 which partially replaces the native valve function. The valve 40 will close off that part of the aperture of the native valve that is not closed by the native leaflets.

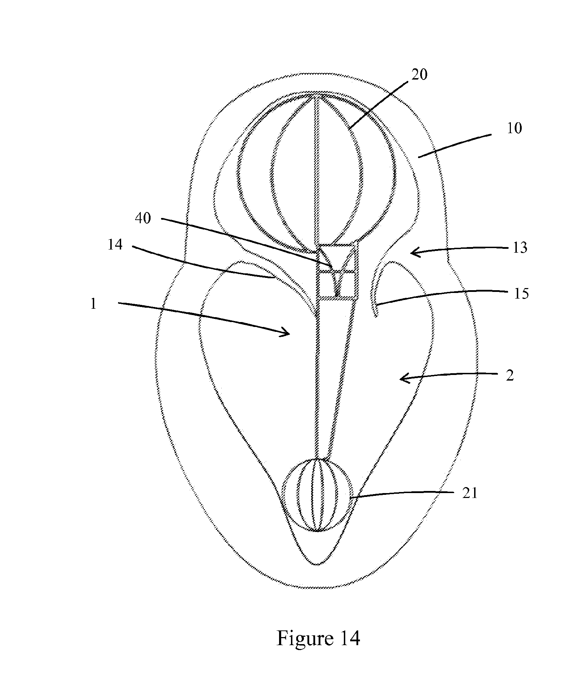

**Figure 14** shows a schematic representation of a device 1 similar to that shown in Figure 2A and 2B wherein the device 1 is configured to be in an offset position where it extends through the native valve 13 of the heart 10 at a position offset to one side of the native valve. In particular the part of the elongate body 2 which runs between the (upper) cage structure 20 and the (lower) cage structure 21 is offset to one side. As can be seen from the Figure the elongate body 2 is configured to be offset to one side (in the embodiment to the right side from the perspective of a viewer in looking at the Figure). This is achieved by having a non-symmetrical device. In such an arrangement the elongate body is configured to pass through a cross-sectional area of the valve which is to a substantially greater extent overlapping with the area of one leaflet as compared to the other. It will be appreciated that the valve member 40 can then be positioned by the device 1 to wholly supplement or replace one leaflet (for example leaflet 15 in the Figure) or simply to do so to an extent that is greater for one leaflet than the other.

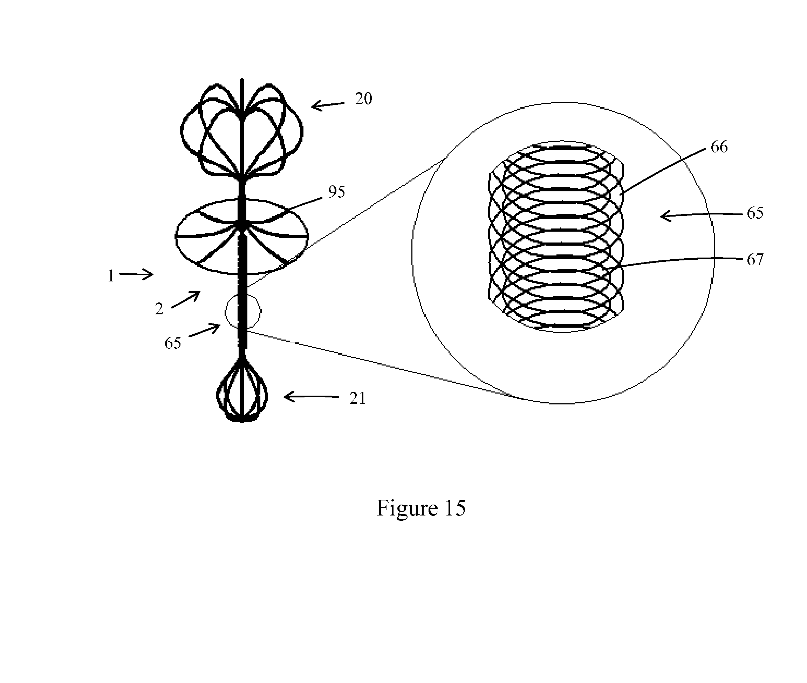

**Figure 15** is a perspective view of a device 1 similar to that shown in Figure 13 further including a damper arrangement 65 for damping axial compression of the device. The arrangement has a similar function to damper 45 from earlier embodiments. It is constructed with upper and lower cage structures 20;21 and an elongate body 2. It has a peripheral skirt 95 for preventing prolapse.

The damper arrangement 65 is shown in an enlarged view also where it can be seen that the damper comprises two helical coils, and outside coil 66 and an inside coil 67. The two coils are resiliently deformable under compression (and indeed tension) and thus act as a damper for the elongate body 2. This means that compressive shortening forces experienced by the elongate body 2, for example because of force exerted by the heart on the cages 20,21 can be absorbed by (axial) damping movement of the damper 65.

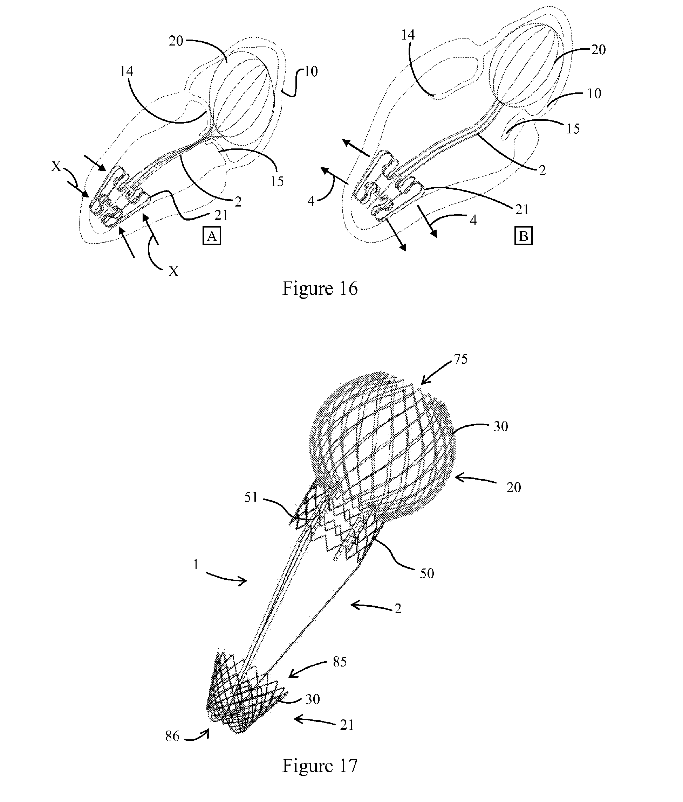

**Figure 16** is a schematic representation of a device 1 of the present invention similar to that of earlier Figures. It has a cantilever lower cage structure 21 which will be described in detail below. In **Figure 16A** **the** cage structure 21 has a compressed configuration because of left ventricle systole (indicated by arrows X) and in **Figure 16B** an expanding configuration during diastole (indicated by arrows Y). In Figure 16A the native valve 13 is closed with leaflets 14, 15 closed and gripping elongate body 2. In this configuration the (resiliently compressible) elongate body 2 has been compressed by the action of the native leaflets 14,15 and thus has a reduced cross-sectional area. During diastole, as shown in Figure 16B the leaflets 14,15 have opened and elongate body 2 has been released returning to its uncompressed state (with a greater cross-sectional area).

It will be appreciated, that the device of the invention being resiliently compressible can compress as shown. It can change shape due to forces imparted to it by the heart and in particular absorb contraction forces imparted by the heart yet resiliently return when the heart moves toward its uncontracted configuration. The device can deal with different forces experienced in different chambers of the heart.

As illustrated by this embodiment the device of the invention can have a bent shape when implanted, such as where the elongate body is curved. The bent shape can be configured in the original device or adopted in response to forces imparted by the heart once implanted and/or the shape can be present during diastole or systole or during just one phase for example systole.

**Figure 17** is a schematic representation of a device 1 of the present invention similar to that of earlier Figures with different resiliently compressible parts, in particular the (upper) cage structure 20 and the (lower) cage structure 21 are differently configured.

For example the cages 20 and 21 takes a form similar to that of a stent construction where there is a mesh arrangement of struts 30 forming each cage. The cage 20 is bulbous in shape similar to earlier embodiments but has an open top or crown 75 which forms an aperture across which the struts 30 do not extend. A ring or band 50 of a compressible lattice structure formed by struts 51 is held on the device and is generally as described above.

The (lower) cage structure 21 has a tapered profile and forming a tapered basket or cup shape. It has an open top end 85 and an open bottom end 86. Such alternative shapes can be utilised for better physiological fit with the heart.

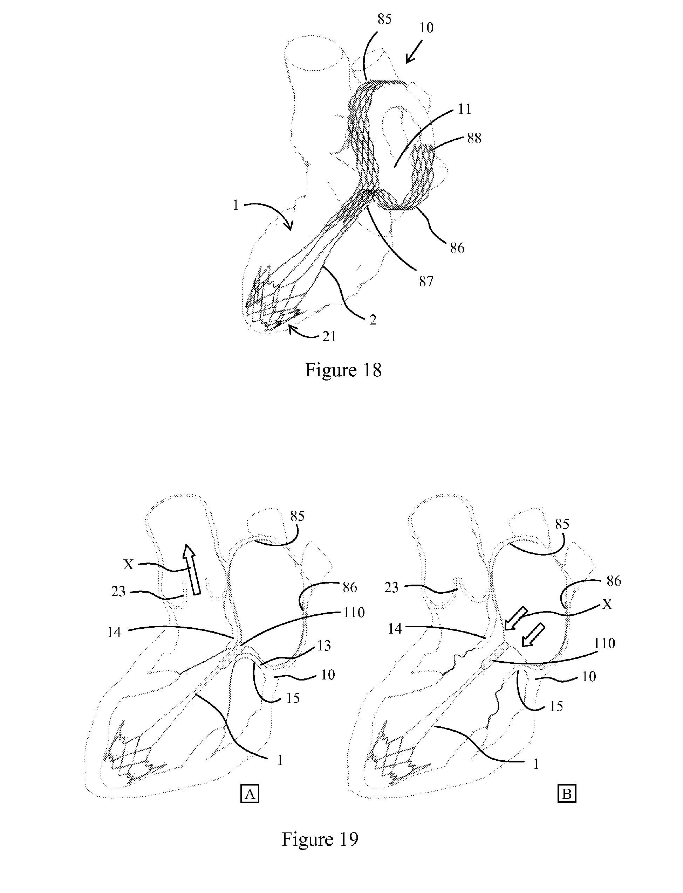

**Figure 18** is a perspective view of a device of the invention that is similar to that of Figure 17 but with a different resiliently compressible upper part 20. The upper part 20 comprises a compressible lattice structure with a curved outer profile. In particular the upper part 20 comprises two opposing arms in the form of leaves 85,86 which are resiliently biased apart and are adapted to be compressively engaged by the heart 10. The leaves 85,86 respectively press against the inner anterior and posterior surfaces of the atrium 11. The leaves 85,86 are curved, for example in cupped shape each forming a partial C-shape and together forming an overall substantially c-shaped configuration. The leaves 85,86 join together at a hinge or join 87 on the elongate body 2. They are resiliently biased apart at the hinge 87. As the heart contracts the leaves move toward (systole phase of rhythm) and away (diastole phase of rhythm) from each other. The upper compressible part of a device of the invention can be formed by a forked structure. It can be bifurcated as in the Figure 18 embodiment or branched off three or more times. Each leaf 85,86 has a lattice structure formed by struts 88. It will be appreciated that the open lattice structures utilised in the invention allow for throughflow of blood. The leaves can be joined together for example at a top end thereof, such as by a joining member but desirably still be moveable toward each other by heart action. This can add additional stability to the device.

**Figure 19** is a sectional view of a device of Figure 18 additionally configured as a plugging device, shown in **Figure 19A** in systole and **Figure 19B** in diastole. The device 1 differs from that in Figure 18 by having a plug 110 which is intended to supplement the action of the mitral valve 13 as it plugs a gap between the leaflets 14,15 of the mitral valve 13 during ventricle systole (Figure 19A) when mitral valve 13 is closed and aortic valve 23 is open. It can thus help prevent leakage due to incorrect closing of the valve 13 as it provide additional blocking of bloodflow when the native valve 13 is closed. Figure 19B shows the diastole phase with leaflets 14,15 of valve 13 open and the aortic valve 23 closed. Blood flow direction is indicated by arrow(s) X in each case.

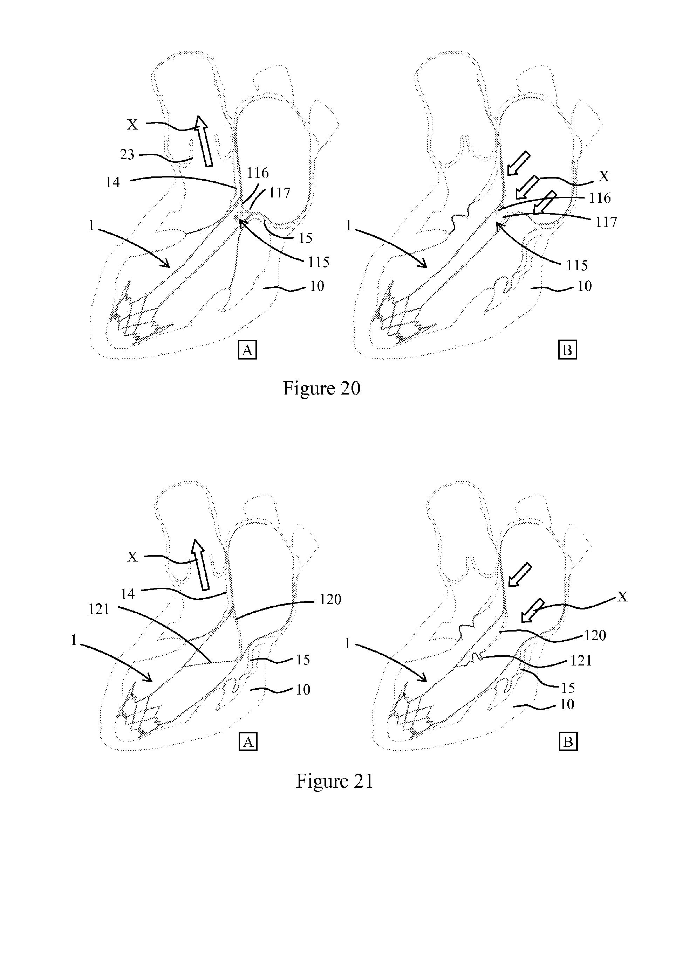

**Figure 20** is a sectional view of a device of Figure 18 additionally configured with a supplementing prosthetic valve 115, shown in **Figure 20A** in systole and **Figure 20B** in diastole. The prosthetic valve 115 is configured to supplement mitral valve 13. It is configured to open and close with the pumping cycle of the heart 10. In Figure 20A when the mitral valve 13 is closed leaflets 116,117 of prosthetic valve 115 are also closed. Leaflets 14,15 of mitral valve 13 close about the prosthetic valve 115 so that together the prosthetic valve 115 and the mitral valve 13 close during left ventricle systole. Blood flow is then out through aortic valve 23. Figure 20A shows the position when left ventricle diastole occurs with both the mitral valve 13 and the prosthetic valve 115 open with blood flow out though the mitral valve. Blood flow direction is indicated by arrow(s) X in each case.

**Figure 21** is a sectional view of a device of Figure 18 additionally configured with a supplementing valve leaflet 120, shown in **Figure 21A** in systole and **Figure 21** **B** in diastole. To mimic the natural heart tendons (chordae tendineae) the leaflet 120 is provided with a prosthetic tendon 121 that limits the movement of the leaflet to avoid prolapse. The prosthetic leaflet 120 is arranged to effectively replace the posterior leaflet 15 which effectively remains in an open position throughout the systole and diastole cycles (see both Figure 21A and Figure 21B). In Figure 21A the prosthetic leaflet 120 is closed and effective closes the part of the natural valve 13 otherwise closed by (natural) leaflet 15, while the second (natural) leaflet 14 closes off the remainder of the valve aperture by closing against the device 1 forming a closed configuration as shown in Figure 21A.

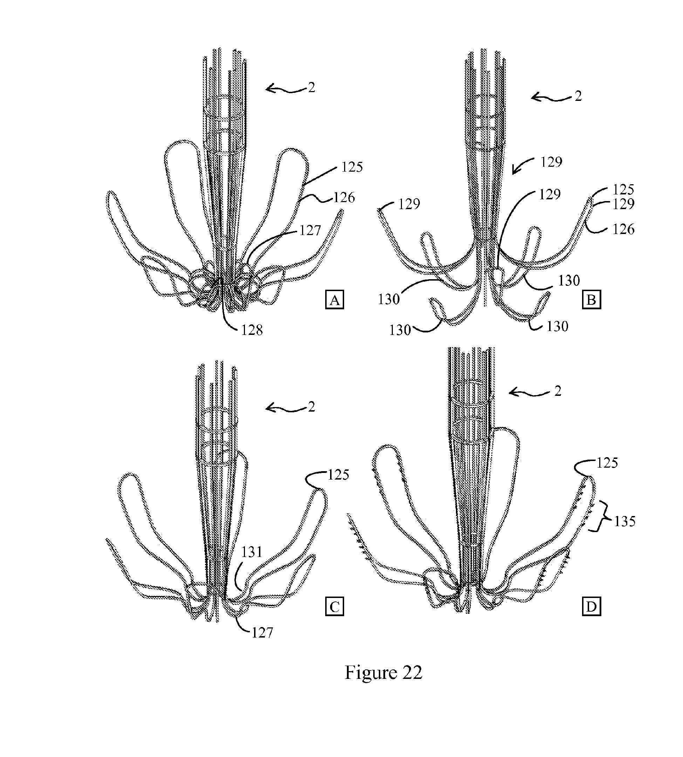

**Figures 22 A-D** are schematic representations of structures suitable for forming a first or second compressible part of a device of the invention and in particular in the form of resiliently biased leaves 125. In the embodiment the resiliently biased leaves form a lower compressible part 21 of a device of the invention.

**Figure 22A** shows an embodiment with six leaves 125 circumferentially distributed about the elongate body 2. The leaves 125 are each formed in a lever type shape by an elongate hoop of ring 126. Each of the leaves 125 have an articulation point 127 which joins it to the elongate body 2 of the device 1 of the invention (the remainder of the device 1 has been omitted for the purposes of illustration). The leaves 125 can resiliently articulate about articulation points 127. Smaller rings, or spring revolutions, 128 form the articulation points 127. The hoop or ring 25 forming the leaf, and the smaller ring(s) 128 can each be formed by bending a suitable rod of material into shape. The leaves 125 are resiliently biased against moving relative to the elongate body 2 and will bias back toward the (rest) position shown in Figure 22A when force is removed. Such an arrangement with a suitable no. of leaves can be considered a cantilever structure. It will be appreciated that the cantilever structure is rounded both in terms of ends/edges but also in profile, again to minimise the chances of damage to the heart when the heart muscle grips the leaves 125.

**Figure 22B** shows an embodiment which is very similar to that shown in Figure 22A where the leaves are vertically spaced from each other. There are eight leaves 125 in a cantilever arrangement. The arrangement is simpler with no rings (such as rings 128 described above) to form articulation points. Instead relative movement of the leaves 125 and the elongate body 2 is allowed by resilient deformation of the leaves 125. In this arrangement however there are two groups each of four leaves which are vertically spaced from each other on elongate body 2. There is a set of (upper leaves) 129 vertically positioned above a second set of (lower) leaves 130.

**Figure 22C** shows an embodiment which the same as that shown in Figure 22A where leaves 125 can resiliently articulate about articulation points 127. A half turn or half spring revolutions 131 form the articulation points 127. As above the half spring revolutions 131 can be formed by bending a suitable rod of material into shape.

**Figure 22D** shows an embodiment which the same as that shown in Figure 22C but with barbs 135 thereon to help secure the device in place in the heart by gripping the heart wall. This ameliorates potential unwanted movement of the device after implantation.

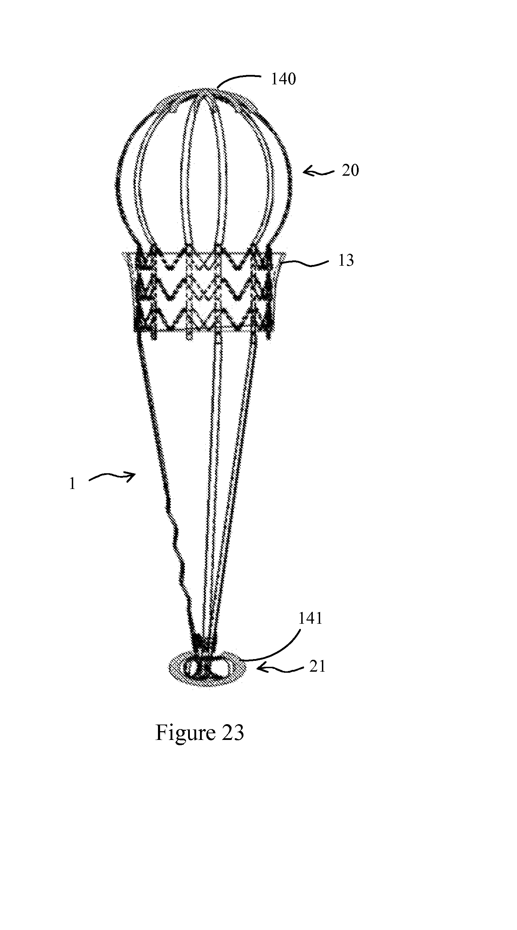

**Figure 23** shows a device 1 of the invention in an arrangement very similar to that of Figure 10 (within the natural valve 13) and with cushioning buffers or barriers on the device. In particular the upper 20 and lower 21 parts of the device are fitted with cushioning buffers or barriers. There is a cushion 140 of material which forms a pad or buffer between the device and the wall of the heart to ameliorate abrasion of the heart by the device 1. It allows for wider distribution of any force exerted between the heart 10 and the device 1. Desirably the cushion is formed by a material which promotes the ingrowth/integration of heart tissue and it may be treated, for example coated or impregnated with growth promoters. Suitably the material is fibrous as it will then have apertures which allow better integration.

A similar cushion or buffer is shown at the lower part of the device and it too can have the function of cushion 140. In this case it takes the form of a circumferentially extending ring 141 which can act as a cushion about the lower part of the device.

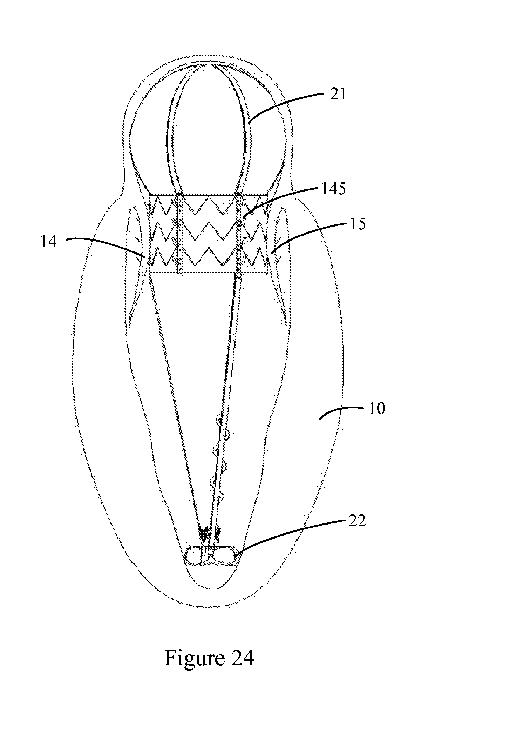

**Figure 24** shows a device of the invention in an arrangement very similar to that of Figure 10 and with barbs 145 attached to the device at a position which engages the mitral valve 13 and in particular the annulus of the mitral valve. This arrangement may be used with any of the valve members described previously. In particular providing the barbs 145 allows for resistance to forces that may otherwise push the device out of position. In particular this arrangement will allow the part of the heart about the mitral valve to absorb some forces that could otherwise be transmitted to the atrium chamber. The atrium is relatively thin-walled and it is desirable to redistribute any load it may experience in this way. The barbs can be used to catch the annulus of the native valve, one or both leaflets of the valve, and/or the heart wall. Where it is attached to the heart wall it is desirably attached to the wall of the ventricle chamber.

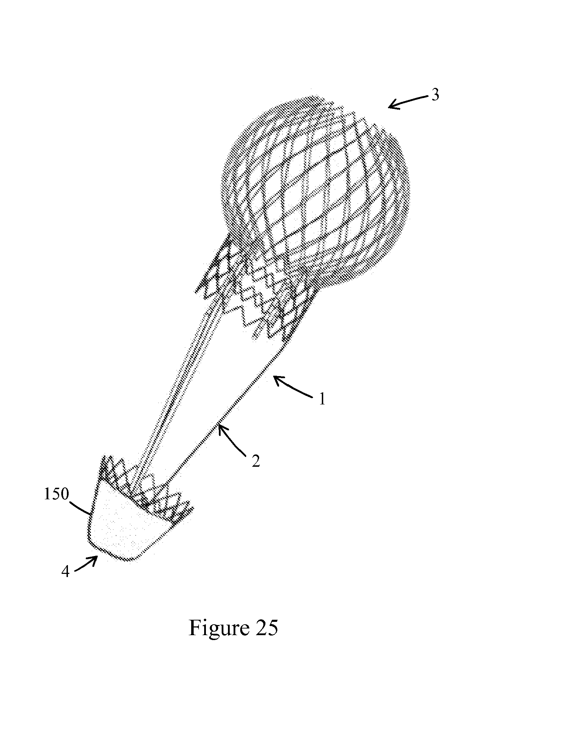

**Figure 25** is a schematic representation of a device 1 of the invention in the form of a prosthesis with a treatment element for reducing the volume of a chamber of the heart. The device 1 may have one or more further treatment elements which are the same or different for example of any type hereinbefore described.

The device 1 is of the type shown in Figure 17 and has a treatment implement which is a volume-reducing component in the form of a partition membrane 150 which desirably takes a concave shape as shown in the figure. The membrane 150 is positioned at the lower end 4 of the device and is dimensioned to extend across the heart to occlude part of a chamber of the heart such as a space 151 (see Figure 2C) between the apex 18 of a ventricle 12. This means the device can reduce the effective blood-receiving volume of a chamber such as ventricle 12. In the embodiment the partition membrane 151 is at the lower end 4 of the device but it will be appreciated that it can be positioned on the upper end 3 or at any position desired. It will also be appreciated that the shape and size of the partition can be designed to effect a desired volume reduction. A partition membrane can be positioned on each end of the device for use in chamber on opposing sides of a native valve. The device may additionally include one or more of any of the treatment elements described above, such as a valve member to replace or repair a native valve.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. An implant device for a heart having a first chamber and a second chamber and a native valve between the chambers, the device comprising:
a. an elongate body having opposing ends, the body being configured to be implanted within the heart and extend through the native valve with a first end in the first chamber and a second end in the second chamber;
b. a first resiliently compressible part of the body being adapted to be compressively engaged by the heart within the first chamber; and
c. a second resiliently compressible part of the body being adapted to be compressively engaged by the heart within the second chamber;
so that the first and second parts hold the elongate body in a desired position within the heart by said compressive engagement.

2. A device according to Claim 1 further comprising at least one treatment element for treating the heart.

3. A device according to Claim 2 wherein at least one treatment element is a valve member and the device is adapted to position the valve member in the heart proximate the native heart valve.

4. A device according to Claim 3 wherein the valve member is selected from one or more of:
a. a replacement valve for replacing the native valve;
b. a replacement leaflet for replacing a native leaflet;
c. a supplementary valve for supplementing the native valve;
d. a stop member for restricting movement of at least one leaflet of the native valve;
e. an occlusion member for occluding an aperture such as an aperture in a valve, or a gap not closed by the valves.

5. A device according to Claim 3 or Claim 4 wherein the valve member:
is collapsible for delivery of the device; and/or
is resiliently compressible so that it is compressible by the native valve; and/or
is dimensioned so as to be under radial compression by the native valve when in place in the heart, and/or
further comprises an occluding peripheral skirt for occlusion of blood flow past the valve member in at least one direction.

6. A device according to any of Claims 3 to 5 wherein the valve member further comprises replaceable leaflets.

7. A device according to any of Claims 2 to 6 wherein at least one treatment element comprises a volume-reducing component adapted to form a partition within a chamber to reduce the effective volume of the chamber.

8. A device according to any of the preceding claims wherein at least one of the first part or the second part comprise a series of struts; and/or
wherein at least one of the first part or the second part comprises a compressible lattice structure; and/or
wherein at least one of the first part or the second part comprises a periphery that forms a loop about a longitudinal axis of the elongate body; and/or wherein at least one of the first part and the second part is formed by at least one, and desirably at least four closed compressible loops.

9. A device according to any of the preceding claims wherein at least one of the first part or the second part comprises at least two leaves which are resiliently biased part and are adapted to be compressively engaged by the heart.

10. A device according to any of the preceding claims wherein at least one of the first part or the second part comprises a compressible cage structure adapted to be compressively engaged by the heart.

11. The device according to any of the preceding claims wherein the elongate body is collapsible to a delivery configuration; and/or
wherein the body is cut from a tubular body; and/or
wherein the body is constructed from at least one braided filament; and/or wherein at least one of the first part or the second part is dimensioned to substantially occupy a chamber.

12. A device according to any preceding claim further comprising a cushioning barrier for cushioning between the device and heart tissue; and/or
tissue integration material attached to at least one of the first or second parts and adapted to promote tissue growth over the device; and further optionally wherein the cushioning barrier is formed by tissue integration material adapted to promote tissue growth over the device;
and/or wherein the cushioning barrier and/or tissue integration material additionally functions to form a partition of a chamber to reduce the effective volume of the chamber.

13. A device according to any preceding claim further comprising one or more barbs for anchoring the device to the heart, optionally wherein the barbs are on the device on at least one of: the first part; the second part; or where the device comprises a valve member, at a position on or proximate to the valve member, for example wherein the barbs anchor the device to at least one of: native valve leaflet(s) or an annulus of the native valve.

14. A device according to any preceding claim wherein the device is configured to be in an offset position where it extends through the native valve at a position offset to one side of the valve; and/or comprises a treatment member such as a valve member configured to be in an offset position where it extends through the native valve at a position offset to one side of the valve.

15. A device according to any preceding claim wherein the device is provided with a plurality of resiliently compressible parts for a given chamber of the heart, for example two or three cage structures for a chamber of the heart such as the atrium chamber and optionally wherein the elongate body is forked.

16. An implant device for a heart having a first chamber and a second chamber and a native valve comprising native leaflets between the chambers, the device comprising:
an elongate body having opposing ends, the body being configured to be implanted within the heart and extend through the native valve with a first end in the first chamber and a second end in the second chamber,
wherein the device is configured to replace one leaflet of the native valve which leaflet is adapted to work in cooperation with a remaining leaflet of the native valve to achieve native valve-like functionality.

17. An implant device for a heart having a first chamber and a second chamber and a native valve between the chambers, the device comprising:
a. an elongate body having opposing ends, the body being configured to be implanted within the heart and extend through the native valve with a first end in the first chamber and a second end in the second chamber;
b. a first part of the body being adapted to be engaged within the first chamber; and
c. a second part of the body being adapted to be engaged within the second chamber;
so that the first and second parts hold the device in an offset position so that it extends through the native valve at a position offset to one side of the valve.
